# EUROPÄISCHE PATENTSCHRIFT

(11) **EP 0 693 930 B1**
(45) Veröffentlichungstag und Bekanntmachung des Hinweises auf die Patenterteilung: **14.10.1998**
(21) Anmeldenummer: 94913533.9
(22) Anmeldetag: 05.04.1994
(51) Int. Cl.: A61K 33/00

(54) **VERWENDUNG VON KOLLOIDALEM SILICIUMDIOXID ZUR BEHANDLUNG DER SICHELZELLANÄMIE, DER MALARIA SOWIE EXOGEN INDUZIERTER LEUKOPENIEN**
USE OF COLLOIDAL SILICON DIOXIDE FOR TREATING SICKLE-CELL ANEMIA, MALARIA AND EXOGENOUSLY INDUCED LEUKOPENIAS
UTILISATION DE DIOXYDE DE SILICIUM COLLOIDAL EN THERAPIE DE L'ANEMIE A HEMATIES FALCIFORMES, DU PALUDISME ET DE LEUCOPENIES A INDUCTION EXOGENE

(30) Priorität: 07.04.1993 DE 4311546
(43) Veröffentlichungstag der Anmeldung: 31.01.1996
(73) Patentinhaber: Schmidt, Alfred, 22301 Hamburg (DE); BISSE, Emmanuel, D-79211 Denzlingen (DE); WIELAND, Heinrich, 79271 St. Peter (DE)
(72) Erfinder: Schmidt, Alfred, 22301 Hamburg (DE); BISSE, Emmanuel, D-79211 Denzlingen (DE); WIELAND, Heinrich, 79271 St. Peter (DE)
(74) Vertreter: Glawe, Delfs, Moll & Partner
(86) Internationale Anmeldenummer: EP9401056
(87) Internationale Veröffentlichungsnummer: WO9422456

(56) Entgegenhaltungen:
- EP-A- 0 126 012
- WO-A-82/03770
- CHEMICAL ABSTRACTS, vol. 83, no. 19, 10. November 1975, Columbus, Ohio, US; abstract no. 159997, IVANOV, Z. ET AL 'Peripheral blood changes induced by the chronic effect of radon and silicon dioxide (in combination or separately)'

## Beschreibung

Die Erfindung betrifft die Verwendung von kolloidalem Siliciumdioxid zur Behandlung der Sichelzellanämie, der Malaria sowie exogen induzierter Leukopenien.

Die Erfindung beruht unter anderem auf der Erkenntnis, daß die orale Gabe von Siliciumdioxid in kolloidaler Form vorteilhaft zur Behandlung von reinerbigen Patienten mit Sichelzellanämie eingesetzt werden kann. Weiterhin bewirkt die orale Verabreichung von Siliciumdioxid eine Normalisierung des Blutbildes, unter anderem unter Stimulierung der Hämatopoese (Blutbildung).

Störungen der Hämatopoese können auf vielfältigem Wege entstehen; im Sinne der Erfindung sind diese Störungen insbesondere hervorgerufen durch Sichelzellanämie und durch Malaria. Exogen induzierte Leukopenien entstehen insbesondere als Folge der Einwirkung von ionisierenden Strahlen sowie durch die Behandlung mit Medikamenten wie Cytostatika.

Zur Zeit wird die Sichelzellanämie durch Bluttransfusionen behandelt. Bei dieser Therapie kommt es im Durchschnitt alle drei Monate zu einer hämolytischen Krise. Diese besteht zum einen in einer sehr starken intraversalen Hämolyse und darüber hinaus in einer Verstopfung der Arteriolen und Kapillaren, die sehr schmerzhaft ist und zu Gewebeschädigungen in den nachgeschalteten Geweben führt. Ein wesentlicher Nachteil dieser Therapie ist, daß der Organismus der Patienten mit Eisen überladen wird. Die Lebenserwartung der Patienten beträgt ca. 30 Jahre. Bei etwa der Hälfte der Kinder kommt es innerhalb von zwei Monaten zu zwei bis fünf hämolytischen Krisen.

Mit dem Gegenstand der Erfindung gelingt es gezielt, die Gesamtzahl der hämolytischen Krisen bei reinerbigen Patienten mit Sichelzellanämie zu verringern; weiterhin können auch Störungen einer auf anderem Wege induzierten Hämatopoese beseitigt sowie exogen induzierte Leukopenien behandelt werden.

Der Wirkungsmechanismus des Siliciumdioxids bei der Sichelzellanämie ist nicht bekannt. Möglich ist eine Hemmung der Polymerisierung des bei dieser Krankheit abnormen Hämoglobins. Denkbar ist weiterhin auch eine Stabilisierung der Erythrocytenoberfläche mit der Folge, daß die Bildung von Sichelzellen ausbleibt und damit die hämolytischen Krisen vermieden und die Gewebe ausreichend perfundiert werden. Weiterhin ist auch eine Verbesserung des Sauerstofftransports durch das Blut infolge der Pufferwirkung der Kieselsäure denkbar, durch die der intrazelluläre pH-Wert der Erythrocyten positiv beeinflußt wird.

In einer kontrollierten Untersuchung von an Sichelzellanämie erkrankten Patienten konnten mittels der erfindungsgemäßen Verwendung bei den Patienten auftretende hämolytische Krisen erheblich vermindert werden. 90% der Kinder wiesen nur noch höchstens eine Krise alle zwei Monate auf; lediglich 10% der Kinder erlitten eine Krise pro Monat. Hierdurch konnte die Anzahl der erforderlichen Bluttransfusionen in der untersuchten Gruppe ebenso wie die Häufigkeit des Krankenhausaufenthaltes drastisch verringert werden. Der Prozentsatz der Kinder, die entweder ins Krankenhaus aufgenommen mußten oder zwei bis dreimal innerhalb von zwei Monaten eine Bluttransfusion erhielten, konnte von 20% auf 0% gesenkt werden. Die erfindungsgemäße Verwendung führte gleichzeitig zu einer deutlichen Verminderung der Begleitsymptome der Sichelzellanämie. Das Ausmaß der Gelbsucht, die Vergrößerung der Milz und die Knochenmarkentzündungen gingen bei der Behandlung erheblich zurück.

Für die durchgeführten Untersuchungen wurde das Arzneimittel in kolloidaler Form gegeben; es enthielt erfindungsgemäß 2,8% Siliciumdioxid; derartige Präparationen von kolloidalem Siliciumdioxid sind im Handel erhältlich. Erfindungsgemäß wurde das Mittel in einer Menge von 2 x 1 Eßlöffeln pro Tag, gelöst bzw. suspendiert in etwas Mineralwasser, vor den Mahlzeiten gegeben.

Der Effekt des erfindungsgemäß eingesetzten Mittels wurde in Abhängigkeit von der Behandlung und Zeit untersucht, und zwar insbesondere im Hinblick auf
1. die Anzahl der hämolytischen Krisen innerhalb von drei Monaten und
2. die Veränderungen der Parameter des roten und weißen Blutbildes.

Es wurden 20 Kinder mit Sichelzellanämie untersucht. Davon dienten 10 unbehandelte Kinder als Kontrollgruppe. Als weitere Kontrolle dienten die behandelten Kinder, bevor sie mit dem erfindungsgemäß eingesetzten Mittel behandelt wurden. Die Verteilungen von Alter und Geschlecht in den einzelnen Gruppen sind aus den Tabellen 1A und 1B ersichtlich.

Zu Beginn der Untersuchung wurde bei jedem Kind ein Differentialblutbild angefertigt und eine klinische Untersuchung durchgeführt, bei der die Behandlung und die Anzahl der vorangegangenen hämolytischen/vaso-okklusiven Krisen erfragt wurde. Diese ist in den linken zwei Dritteln der Tabelle 2A dargestellt.

Die gesamte Untersuchungsdauer betrug 4 Monate. Die Patienten wurden alle 2 bis 4 Wochen untersucht. Dabei wurden die Blutbildparameter bestimmt sowie die Anzahl der Krisen und verabreichte Bluttransfusionen dokumentiert.

Die Tabelle 2a gibt eine Übersicht über die hämolytischen/vaso-okklusiven Krisen in den einzelnen Patientengruppen (Kontrollgruppe und behandelte Gruppe wurden zur gleichen Zeit beobachtet; behandelte Gruppe vor der Therapie, Mittelwerte aus den vergangenen zwei Jahren.

Mit dem erfindungsgemäß eingesetzten Mittel läßt sich die Anzahl der Kinder mit 4 bis 5 oder 2 bis 3 Krisen pro 2 Monaten von 8 bzw. 9 auf 1 Kind reduzieren. Während vor der Therapie nur 2 bzw. 1 Kind lediglich 0 bis 1 Krise alle 2 Monate aufwies, ist dies nun bei 9 Kindern der Fall. Es ergibt sich also eine deutliche Verminderung des Auftretens der Krisen.

Bei der Kontrollgruppe und der behandelten Gruppe mußten vor der erfindungsgemäßen Therapie bei zwei bzw. einem Patienten 2 bis 3 Transfusionen pro 2 Monate gegeben werden. Nach der Therapie lagen alle Patienten in der Gruppe, die keine bis eine Transfusion alle 2 Monate benötigte; vgl. Tabelle 2B.

In Tabelle 3 sind die Häufigkeit der Begleitsymptome der Sichelzellanämie, die Anzahl der benötigten Bluttransfusionen (V5) und die Anzahl der hämolytisch/vaso-okklusiven Krisen (V6) vor und während des erfindungsgemäßen Einsatzes für jeden untersuchten Patienten dargestellt. Diese Symptome sind:
1. Ikterus (V1), 2. Splenomegalie (V2), 3. Osteomyelitis (V3), 4. Infektionen (V4).

Der erfindungsgemäße Einsatz des Mittel führte zu einer statisch hochsignifikanten Verbesserung des alle Symptome umfassenden klinischen Bildes. Er führte mit einer 36-fach erhöhten Wahrscheinlichkeit zur Verminderung der Häufigkeit und der Ausprägung der Symptome (p = 0,0016).

Für die in der Anlage beigefügten Tabellen 1A, 1B, 2A, 2B und 3 gelten die folgenden Legenden:

### Tabelle 1A, 1B:

Einteilung der Patienten nach Alter und Geschlecht. GE: Geschlecht; K: Kontrollgruppe; P: behandelte Gruppe.

### Tabelle 2A:

Wirkung des Mittels auf hämolytisch/vaso-okklusive Krisen in den einzelnen Patientengruppen. AKZ2M = Krisen pro 2 Monate.

### Tabelle 2B:

Wirkung des Mittels auf die Anzahl der benötigten Bluttransfusionen pro 2 Monate (AZT2M).
PO: behandelte Gruppe vor der Therapie; P: nach der Therapie.

### Tabelle 3:

Übersicht über den Symptomverlauf in den behandelten Patienten.
V1: Ikterus; V2: Splenomegalie; V3: Osteomyelitis; V4: Infektionen; V5: Bluttransfusion (+: 2 Transfusionen/2 Monate; ++: 2-3/2 Monate); V6: vaso-okklusive Krisen (-: keine; ± 0-1 Krise/2 Monate; +: 2-3 Krisen/2 Monate; ≥ ++: 4-5 Krisen/2 Monate.)
tn: Zeitraum (n = Monat); -; keine ±: kaum; +: wenig, ++: mäßig, +++: stark.

**Tab.1A**

| | KP | | TP | |
|---|---|---|---|---|
| Altersgruppe | n | % | n | % |
| 4 - 10 | 6 | 60 | 6 | 60 |
| 11 - 20 | 3 | 30 | 2 | 20 |
| 21 - 30 | 1 | 10 | 1 | 10 |
| 31 - 40 | 0 | 0 | 1 | 10 |
| Gesamt | 10 | 100 | 10 | 100 |

**Tab.1B**

| | K | | P | |
|---|---|---|---|---|
| GE | n | % | n | % |
| ♀ | 5 | 50 | 3 | 30 |
| ♂ | 5 | 50 | 7 | 70 |
| Gesamt | 10 | 100 | 10 | 100 |

**Tab.2A**

| | K | | P0 | | P | |
|---|---|---|---|---|---|---|
| AZK2M | n | % | n | % | n | % |
| 0 - 1 | 02 | 20 | 01 | 10 | 09 | 90 |
| 2 - 3 | 04 | 40 | 05 | 50 | 01 | 10 |
| 4 - 5 | 04 | 40 | 04 | 40 | 00 | 00 |
| Gesamt | 10 | 100 | 10 | 100 | 10 | 100 |

**Tab.2b**

| | K | | P0 | | P | |
|---|---|---|---|---|---|---|
| AZT2M | n | % | n | % | n | % |
| 0 | 0 | 00 | 0 | 00 | 8 | 80 |
| 1 | 0 | 00 | 1 | 10 | 2 | 20 |
| 2 | 8 | 80 | 8 | 80 | 0 | 00 |
| 2 - 3 | 2 | 20 | 1 | 10 | 0 | 00 |
| Gesamt | 10 | 100 | 10 | 100 | 10 | 100 |

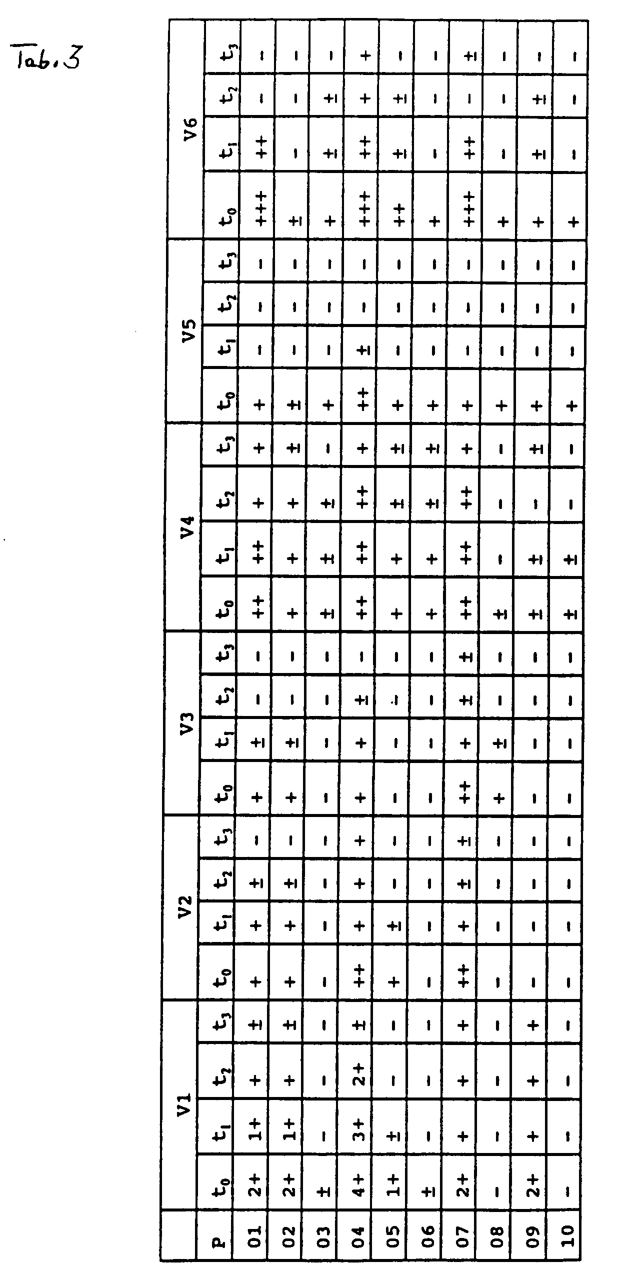

## Patentansprüche

1. Verwendung von kolloidalem Siliciumdioxid zur Herstellung eines Arzneimittels zur Behandlung der Sichelzellanämie, der Malaria sowie exogen induzierter Leukopenien.

2. Verwendung nach Anspruch 1 zur Herstellung eines Arzneimittels zur oralen Verabreichung.

3. Verwendung nach Anspruch 1 oder 2, dadurch gekennzeichnet, daß die Hämatopoese durch eine Sichelzellanämie bei homozygoten Patienten gestört ist.

4. Verwendung nach Anspruch 1 oder 2, dadurch gekennzeichnet, daß die Hämatopoese durch Malaria gestört ist.

5. Verwendung nach Anspruch 1 oder 2, dadurch gekennzeichnet, daß die Leukopenien durch Einwirkung von ionisierender Strahlung oder von Arzneimitteln verursacht sind.

## Claims

1. Use of colloidal silica for the manufacture of a medicament for the treatment of sickle-cell anaemia, malaria and exogenously induced leucopenias.

2. Use according to Claim 1 for the manufacture of a medicament for oral administration.

3. Use according to Claim 1 or 2, characterised in that haematopoiesis is impaired by sickle-cell anaemia in homozygous patients.

4. Use according to Claim 1 or 2, characterised in that haematopoiesis is impaired by malaria.

5. Use according to Claim 1 or 2, characterised in that leucopenias are caused by action of ionising radiation or by medicaments.

## Revendications

1. Utilisation de dioxyde de silicium colloidale pour la fabrication d'un médicament pour le traitement de l'anémie falciforme, de la malaria comme les leucopénies induites exogènes.

2. Utilisation selon la revendication 1 pour la fabrication d'un médicament à administration orale.

3. Utilisation selon la revendication 1 ou 2, caractérisée en ce que l'hématopoièse est perturbée dans le cas d'une anémie falciforme chez des malades homozygotes.

4. Utilisation selon la revendication 1 ou 2, caracterisée en ce que l'hématopoièse est perturbée dans le cas de la malaria.

5. Utilisation selon la revendication 1 ou 2, caractérisée en ce que les leucopénies sont induites par l'effet de rayons ionisants ou de médicaments.
